# EUROPEAN PATENT SPECIFICATION

(11) **EP 2 104 461 B1**
(45) Date of publication and mention of the grant of the patent: **09.04.2014**
(21) Application number: 08705502.6
(22) Date of filing: 03.01.2008
(51) Int. Cl.: A61B 17/34, A61B 1/313, A61B 1/00

(54) **ACCESS SHEATH WITH REMOVABLE OPTICAL PENETRATING MEMBER**
ZUGANGSHÜLLE MIT ABNEHMBAREM OPTISCHEM PENETRATIONSELEMENT
GAINE D'ACCÈS ÉQUIPÉE D'UN ÉLÉMENT DE PÉNÉTRATION OPTIQUE AMOVIBLE

(30) Priority: 03.01.2007 US 878483 P
(43) Date of publication of application: 30.09.2009
(60) Divisional application: 14154735.6
(73) Proprietor: Covidien LP, Mansfield, MA 02048 (US)
(72) Inventor: SMITH, Robert, C., Middletown, CT 06457 (US)
(74) Representative: Soames, Candida Jane
(86) International application number: PCT/US2008/000161
(87) International publication number: WO 2008/085918

(56) References cited:
- US-A- 5 522 833
- US-A- 5 609 604
- US-A- 5 685 820
- US-A- 5 713 870
- US-A- 5 817 061
- US-A1- 2003 004 529
- US-A1- 2006 173 479
- US-A1- 2006 226 655

## Description

### 1. Technical Field

The present disclosure relates to an apparatus for accessing an underlying body cavity and, more particularly, relates to a system incorporating an access sheath having an optical penetrating member which is removable through the access sheath subsequent to accessing the body cavity.

### 2. Background of the Related Art

A variety of surgical procedures require accessing an underlying operative site with a sheath or cannula. For example, in an endoscopic procedure, surgery is performed in a hollow viscus of the body through a small incision or through narrow endoscopic tubes (cannulas) inserted through small entrance wounds in the skin. In laparoscopic procedures, surgery is performed in the interior of the abdomen, typically, through a cannula. Historically, endoscopic surgical procedures were primarily diagnostic in nature. More recently as endoscopic technology has advanced, surgeons are performing increasingly complex and innovative endoscopic surgical procedures.

In a laparoscopic surgical procedure, the abdominal cavity is insufflated with a suitable gas, and a trocar is thereafter utilized to puncture the body cavity. The trocar may include an obturator for penetrating the abdominal tissue and a cannula which is coaxially positioned about the obturator. The obturator is removed from the cannula subsequent to penetration of the tissue thereby leaving the cannula within the tissue for reception of laparoscopic instruments and/or a laparoscope required to perform the desired procedure.

US 5 713 870 discloses a penetrating instrument having features on which the preamble of the independent claim is based.

### SUMMARY

According to the invention there is provided a system for accessing underlying tissue as recited in claim 1.

Accordingly, the present disclosure is directed to further improvements in penetrating tissue during surgical procedures such as endoscopic or laparoscopic surgery. In accordance with one embodiment, a system for accessing underlying tissue includes an elongated access sheath defining a longitudinal axis and a penetrating tip releasably mounted to the access sheath and dimensioned for facilitating passage through tissue. The penetrating tip is removable through the access sheath. The penetrating tip may include a transparent region adapted to permit passage of light.

The system further includes an elongated removal member positionable within the access sheath. The elongated removal member may be mounted with respect to the penetrating tip to permit the elongated removal member to remove the penetrating tip through the access sheath subsequent to passage of the penetrating tip through tissue. The elongated removal member is releasably mounted to the penetrating tip. The elongated removal member and the penetrating tip include cooperating structure for effecting releasable coupling of the elongated member to the penetrating tip. The cooperating structure includes one of a bayonet coupling, threaded coupling, tongue and groove coupling and interference coupling.

A sheath housing may be mounted to the access sheath. The sheath housing may include a valve adapted to form a substantial seal about an elongated object passed through the sheath housing.

The system further may include a laparoscope having an illumination system for delivering illuminating light and an imaging system for detecting and transmitting an illuminated image of the surgical object. The laparoscope may be at least partially positioned within the access sheath to permit visualization during advancement of the access sheath and the penetrating tip within the tissue.

### BRIEF DESCRIPTION OF THE DRAWINGS

Preferred embodiments of the present disclosure are described hereinbelow with references to the drawings, wherein:
**FIG. 1** is a perspective view of the surgical system for accessing underlying tissue in accordance with the principles of the present disclosure illustrating an access sheath with a releasable optical penetrating tip, an elongated removal instrument for removing the penetrating tip and a laparoscope;
**FIG. 2** is an enlarged side view in partial cross-section of the distal end of the access sheath and the optical penetrating tip;
**FIG. 3** is a side plan view of an alternate embodiment of the access sheath and the optical penetrating tip;
**FIG. 4** is an enlarged side view in partial cross-section of the distal end of the elongated removal instrument and the optical penetrating tip;
**FIG. 5** is an enlarged side view in partial cross-section of the distal end of an alternate embodiment of the elongated removal instrument and the optical penetrating tip;
**FIG. 6** is an axial plan view of the optical penetrating tip of FIG. 5;
**FIG. 7** is a view illustrating the penetrating tip mounted to the access sheath and with the laparoscope positioned therein to permit visualization during penetration of tissue; and

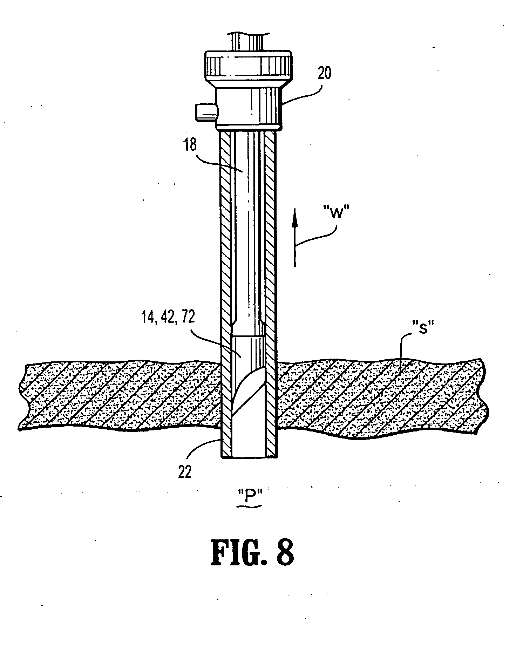

**FIG. 8** is a view similar to the view of **FIG. 7** illustrating the laparoscope removed from the access sheath and the removal instrument introduced within the access sheath to remove the optical penetrating tip.

### DETAILED DESCRIPTION OF THE PREFERRED EMBODIMENTS

The exemplary embodiments of the surgical system and method of use are discussed in terms of accessing an underlying tissue site, particularly, in accessing an underlying body cavity in connection with an endoscopic procedure. However, it is envisioned that the present disclosure may be employed with a range of surgical applications including surgical, diagnostic and related treatments of diseases, body ailments, of a subject.

In the discussion that follows, the term "proximal" or "trailing" will refer to the portion of a structure that is closer to a clinician, while the term "distal" or "leading" will refer to the portion that is further from the clinician. As used herein, the term "subject" refers to a human patient or other animal. The term "clinician" refers to a doctor, nurse or other care provider and may include support personnel.

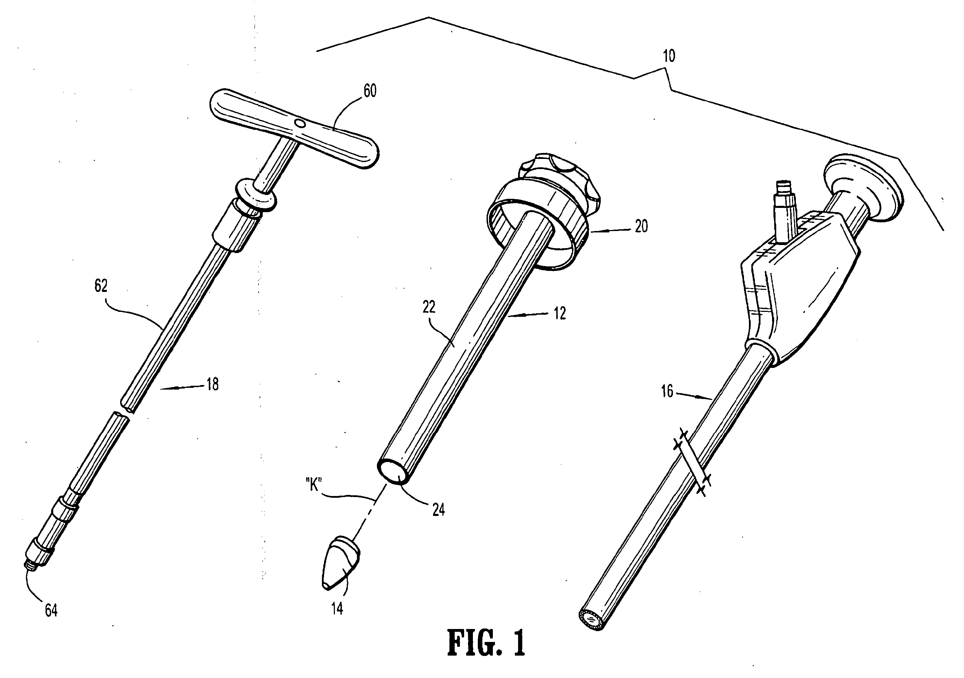

Referring now to the drawings wherein like components are designated by like reference numerals throughout the several views, **FIG. 1** illustrates in perspective view, the surgical system **10** in accordance with the principles of the present disclosure. Surgical system **10** includes several components or instruments, namely, access sheath **12** with associated optical penetrating tip **14,** laparoscope **16** positionable within the access sheath **12** and removal instrument **18.** Access sheath **12** defines a portal for reception of surgical instrumentation such as laparoscope **16,** or any variety of laparoscopic or endoscopic instrumentation including clip appliers, stapling instruments, suturing instruments, graspers, forceps, etc. required to perform the desired surgical procedure. In one embodiment, access sheath **12** is a cannula particularly adapted for use in laparoscopic surgery where the peritoneal cavity is insufflated with a suitable gas, e.g., CO₂, to raise the cavity wall and permit access to the underlying organs. In a conventional laparoscopic procedure, an obturator is positioned within the cannula and utilized to penetrate the abdominal wall. The obturator subsequently is removed from the cannula to permit introduction of the surgical instrumentation utilized to perform the procedure through the passageway.

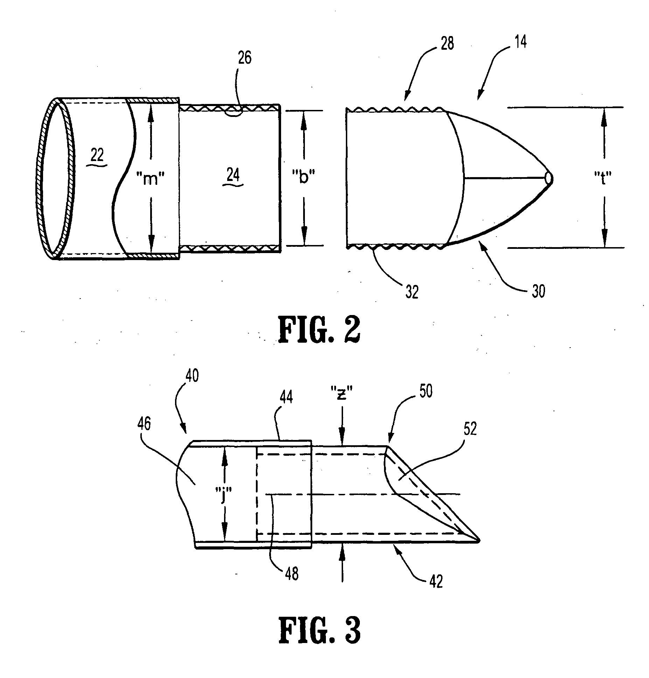

Access sheath or cannula **12** includes access housing **20** and elongated access sleeve **22** extending from the access housing **20.** Access sleeve **22** defines a longitudinal axis "k" extending along the length of the sleeve **22.** Access sleeve **22** further defines internal longitudinal passage **24** dimensioned to permit passage of surgical instrumentation. Access sleeve **22** may be formed of stainless steel or other rigid materials such as a polymeric material or the like. Sleeve **22** may be clear or opaque. The diameter of sleeve **22** may vary, but typically ranges from about 4.5 to about **15** mm. In one application, access sleeve **22** includes internal threaded portion **26** within leading or distal end of the access sleeve **22** to releasably secure optical penetrating tip **14** to the access sleeve **22.** Leading end incorporating threaded portion **26** preferably has a reduced diameter "b" relative to the remainder of the sleeve to define a stepped configuration as best depicted in **FIG. 2****.**

Access housing **20** may include several components connected to each other through conventional means or alternatively may be a single housing component. Access housing **20** may be attached to access sleeve **22** by any suitable means or may be integrally formed with the access sleeve **22.** Access housing **20** may further include an internal zero closure valve which is adapted to close in the absence of a surgical instrument and/or in response to the pressurized environment of the insufflation gases present in the abdominal cavity. One suitable zero closure valve contemplated for use with access housing **20** is a duck bill valve, flapper valve, or the like.

Access housing **20** may also include an internal seal preferably adapted to form a substantial fluid tight seal about an instrument inserted through the seal. One suitable internal seal is a flat disc-shaped valve, balloon valve, etc.... The internal seal may comprise a flat disc-shaped, conical, or hourglass-shaped member including a fabric material molded with an elastomer. The seals disclosed in certain embodiments of commonly assigned U.S. Patent No. 6,482,181, may be used. Seals disclosed in certain embodiments of commonly assigned U.S. Patent Application No. 2004/0066008A1, filed October 4, 2002, may be used. In a further alternative, the internal seal is preferably a fabric seal and is desirably arranged so as to have a constriction. For example, the valve may have the general shape of an hourglass. The fabric can be a woven material, a braided material, or a knitted material. The type of material is selected to provide a desired expensiveness. For example, a braid of varying end count and angle may be selected. A preferred material is a synthetic material such as nylon, Kevlar (Trademark of E.I. DuPont de Nemours and Company) or any other material that will expand and compress about an instrument inserted therethrough. The selected material desirably minimizes or prevents the formation of gaps when the instrument is introduced into the seal. The material of the seal may be porous or impermeable to the insufflation gas. If porous, the seal may include a coating of a material which is impermeable to the insufflation gas or at least a portion of the valve may be coated. In addition, the fabric may be coated on its interior with urethane, silicon or other flexible lubricious materials to facilitate passage of an instrument through the seal. In certain embodiments, the fabric is twisted about the axis "a" so as to form a constriction or closed portion. The fabric is desirably constructed of a material and/or arranged so that the fabric forms a constriction or closure. The seal may also be molded so as to have a constriction or may be knitted, braided or woven so as to have a constriction. Other arrangements for the seal are also envisioned.

Referring now to **FIGS. 1-2****,** optical penetrating tip **14** of the present disclosure will be discussed. Optical penetrating tip **14** is contemplated for mounting to access sleeve **22** to provide access sheath **12** with penetrating capabilities thus obviating the need for a separate obturator introduced within the access sleeve **22.** Optical penetrating tip **14** when mounted to access sleeve **22** is particularly suitable for use with a viewing device such as an endoscope or laparoscope **16** introduced within access sleeve **22.** In this capacity, optical penetrating tip **14** has a transparent region or window to permit direct visualization of body tissue with laparoscope **16** during penetration of the peritoneal cavity or other tissue portions. In one application, optical penetrating tip **14** may have lens surfaces to modify, correct or alter visualization through the optical penetrating tip **14.** Optical penetrating tip **14** is dimensioned to pass through body tissue and may incorporate structure to separate, retract, dissect, cut, puncture, or pierce the body tissue. Such structure is inclusive of cutting edges, blades, points, etc.

Optical penetrating tip **14** includes proximal mounting section **28** and distal penetrating section **30.** Proximal mounting section **28** is generally cylindrical in configuration defining external thread **32** which cooperates with internal thread **26** within access sleeve **22** to connect the two components. Other means for mounting optical penetrating tip **14** to access sleeve **22** are also envisioned including a bayonet coupling, snap fit, tongue and groove mechanism, etc. Some of these methodologies will be discussed hereinbelow. Optical penetrating tip **14** may comprise a polymeric material and be fabricated via known injection molding techniques. Alternatively, optical penetrating tip **14** may comprise an optical glass. In one embodiment, distal penetrating section **30** defines a transparent region or window which permits visualization along the axis "k" of access sleeve **22** and, desirably, locations offset relative to the axis "k". The term "transparent" is to be interpreted as having the ability to permit the passage of light with or without clear imaging capabilities. Moreover, the transparent material includes any transparent or translucent material or any material which is not opaque to visible light or other radiation utilized for imaging. It is also to be appreciated that only a portion of distal penetrating section **30** needs to be transparent. Furthermore, a portion of optical penetrating tip **14** or the entire tip may be translucent or transparent.

Distal penetrating section **30** including the transparent window may define a variety of geometrical configurations including the generally conically arrangement depicted in **FIGS. 1-2****.** Alternatively, distal penetrating section **30** may be pyramidal in configuration having cutting edges, incorporate a dolphin-shaped design or incorporate a cutting blade. The leading end of distal penetrating section **30** may be blunt or pointed. Moreover, distal penetrating section 30 may be intended to penetrate tissue through an incising action or through a more blunt dissecting approach.

Optical penetrating tip **14** preferably defines a maximum outer diameter "t" which is less than the internal diameter of the proximal portion of access sleeve **22** to permit removal of the optical penetrating tip **14** through the access sleeve **22.** Specifically, the outer diameter of external thread **32** and the outer diameter of optical penetrating tip **14** is less than the inner diameter "m" of access sleeve **22** to enable the optical penetrating tip **14** to be removed via removal instrument **18** subsequent to penetration of the body cavity. Moreover, the reduction in the diameter of leading end of access sleeve (having diameter "b") and in the diameter "t" of optical penetrating tip enables unencumbered removal of optical penetrating tip **14** through access sheath **12.** Removal of optical penetrating tip **14** from access sheath **12** will be discussed in greater detail hereinbelow.

**FIG. 3** illustrates an alternate embodiment of access sheath **40** and optical penetrating tip **42.** In accordance with this embodiment, leading end **44** of access sleeve **46** of access sheath **40** is devoid of internal threads and may define an internal diameter "j" which is constant throughout its length. Optical penetrating tip **42** has proximal section **48** which is generally cylindrical and is also devoid of threading. The maximum diameter "z" of optical penetrating tip **48** preferably approximates the inner diameter "j" of access sleeve **46** whereby a close tolerance or frictional fit is established between the two components. With this arrangement, optical penetrating tip **42** is releasably mountable to access sheath **40.** Also, with this embodiment, transparent window **50** of optical penetrating tip **42** defines a lens structure incorporating several lens surfaces **52** to widen or alter the angle of view of laparoscope **16.** For example, transparent window **50** may increase the field of view of laparoscope **16** or, alternatively, may alter the view to provide an inclined angle of view. Other arrangements are also envisioned.

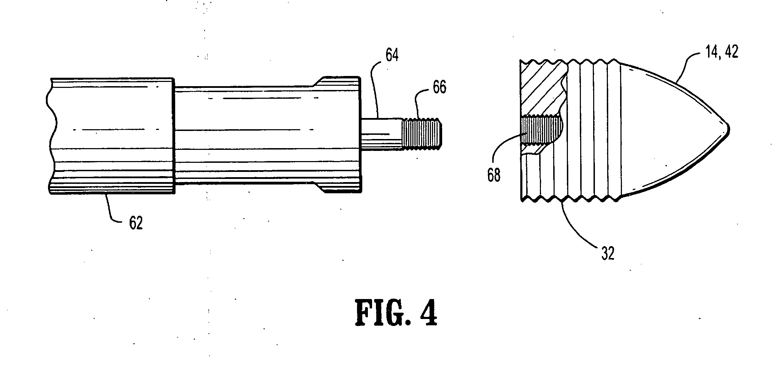

Referring now to **FIG. 4****,** in conjunction with **FIG. 1****,** removal instrument **18** of surgical system **10** will be discussed. Removal instrument **18** includes handle **60** and elongated shaft **62** extending distally from the handle **60**. Handle **60** may be "t-shaped" or any other structure appropriately dimensioned for manual engagement by the clinician. Elongated shaft **62** defines leading end **64** having a reduced diameter with external thread **66.** External thread **66** cooperates with internal thread **68** of optical penetrating tip **14, 42** to releasably mount removal instrument **18** to the optical penetrating tip **14, 42.** In one application, the threads of external thread **66** of removal instrument **18** and the threads of internal thread **68** of optical penetration tip **14** are in the same direction to enable the clinician to connect the removal instrument **18** to the optical penetrating tip **14** and thereafter release the optical penetrating tip **14** from its mounting to access sleeve **22** by rotating handle **60** in the same direction. This also potentially minimizes release of removal instrument **18** from the optical penetrating tip **14.** Other means for coupling removal instrument **18** to optical penetrating tip **44** are also envisioned including a bayonet coupling, tongue and groove arrangement, frictional fit, magnetic relationship, vacuum or suction means, hook and loop means or the like.

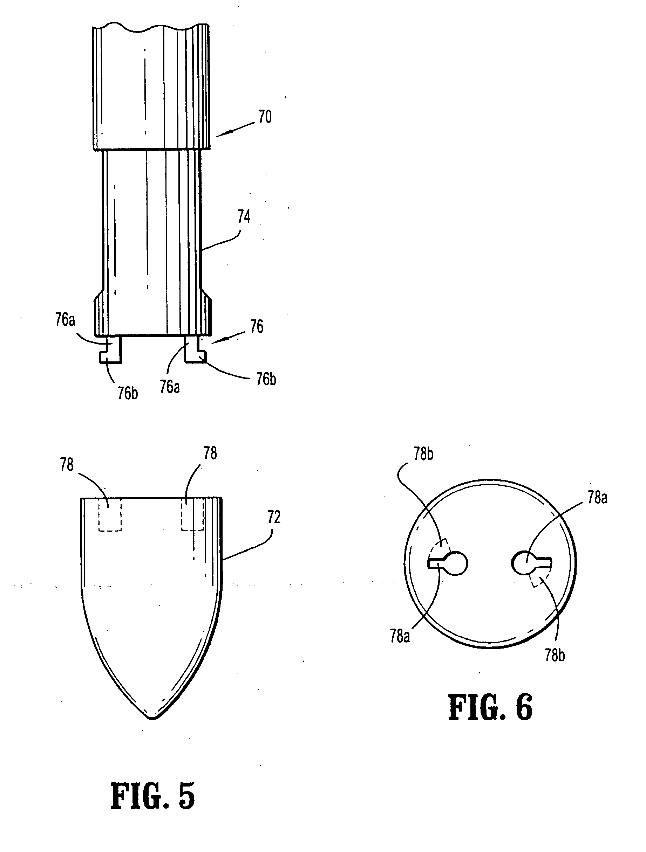

**FIGS. 5** and **6** illustrate an alternate embodiment of removal instrument **70** and optical penetrating tip **72.** In accordance with this embodiment, removal instrument **70** includes leading end **74** with at least one external pin **76,** preferably, two opposed pins **76** extending radially outwardly from the external surface of elongated shaft. External pins **76** are received within corresponding slots **78** within the interior of optical penetrating tip **72** to releasably connect the components through a bayonet coupling relationship. In particular, each pin **76** includes longitudinal pin leg **76**a and transverse pin leg **76**b. Each slot **78** includes corresponding longitudinal slot portion **78**a and transverse slot portions **78**b. Pin **76** is received within longitudinal slot portion **78**a of slot **78** during initial insertion of leading end **74** within optical penetrating tip **72.** Once pins **76** bottom out, removal instrument **70** is rotated whereby transverse pin leg **76**b traverses transverse slot portion **78**b thereby releasably connecting optical penetrating tip **70** to the removal instrument **70.**

Referring again to **FIG. 1****,** laparoscope **16** may be any conventional scope suitable for endoscopic applications including, e.g., a laparoscope, arthroscope, colonoscope, etc. In one preferred embodiment, laparoscope **16** may be the scope disclosed in commonly assigned U.S. Patent No. 5,412,504 to Leiner, the entire contents of which disclosure are hereby incorporated by reference. Laparoscope **16** incorporates an optical train or lens arrangement which is capable of transmitting an image of an object from the distal or objective lens through the eyepiece or monitor for viewing by the surgeon. Further details of laparoscope **16** may be ascertained by reference to the **'504** patent.

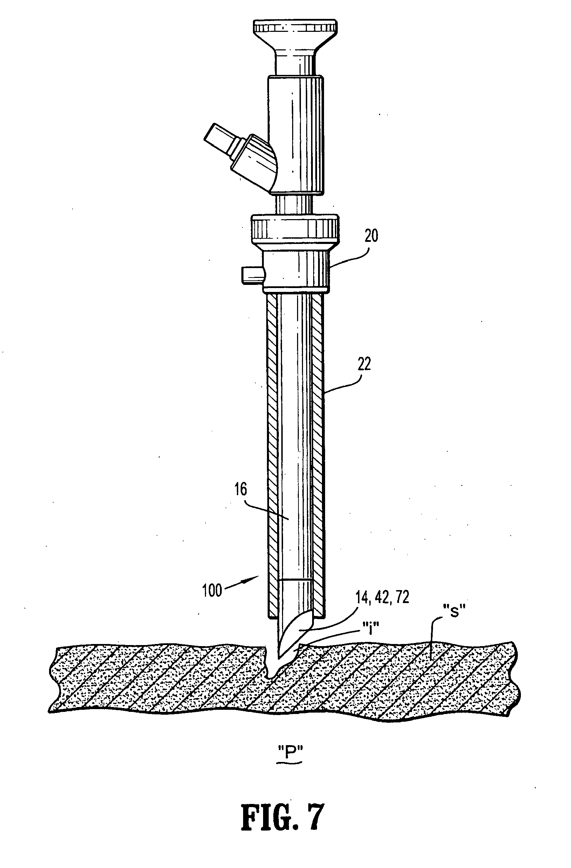

Referring now to **FIG. 7****,** the use of the system **10** during a laparoscopic surgery will be discussed. The peritoneal cavity "p" is insufflated as is conventional to raise the cavity wall to provide greater access to tissue and organs therewithin. Thereafter, any of the aforementioned optical penetrating tips **14,42,72** is mounted to access sleeve **22** of access sheath **12.** Laparoscope **16** is positioned within access sheath **22.** The internal seal within access housing **20** may form a fluid tight seal about the laparoscope **16.** As appreciated, laparoscope **16** is advanced within access sleeve **22** until the distal end of the laparoscope **16** is adjacent the transparent window of optical penetrating tip **14,42,72.** In this position, the distal lens element of the laparoscope **16** is adjacent the transparent window so as to be capable of viewing the tissue being entered. Laparoscope **16** may be secured relative to the access sheath **12** utilizing a resilient washer or a cam locking system incorporated with the access sheath **12** or formed separately therefrom.

The procedure is continued by positioning optical penetrating tips **14,42,72** within a previously formed opening or incision "i" in tissue "s" and advancing the optical penetrating tip **14,42,72** to retract, dissect, or penetrate the tissue. Alternatively, optical penetrating tip **14,42,72** may pierce the tissue to form the incision "i". During penetration of the body tissue, the surgeon observes the underlying tissue through the laparoscope **16** to ensure there is no undesired contact with organs, tissue, etc. lying beneath the peritoneal lining. In instances where a video system is utilized, the surgeon simply observes the penetration of body tissue "s" via any known video monitor. Once the surgeon penetrates the body tissue "s" and positions the distal end of access sleeve **22** in the desired position within the peritoneal cavity "p" as observed through the laparoscope **16,** the surgeon discontinues the application of force.

Laparoscope **16** may then be removed from access sheath **12.** Thereafter, removal instrument **18** is advanced within access sheath **12** and advanced until the leading end is adjacent optical penetrating tip **14,42,72.** Removal instrument **18** is then coupled to optical penetrating tip **14,42,72** in any of the aforementioned manners, which is dependent on the coupling structure. Removal instrument **18** is removed from access sheath **12** as shown by the directional arrows "w" to remove optical penetrating tip **14,42,72** leaving access sheath **12** positioned within the body tissue "s'. Endoscopic instrumentation is advanced within access sheath **12** to perform the desired surgery. Surgery is then carried out through other access sheaths or cannula assemblies which access the peritoneal cavity.

It will be understood that various modifications and changes in form and detail may be made to the embodiments of the present disclosure without departing from the scope of the invention. Therefore, the above description should not be construed as limiting the invention but merely as exemplifications of preferred embodiments thereof. Those skilled in the art will envision other modifications within the scope of the present invention as defined by the claims appended hereto. Having thus described the invention with the details and particularity required by the patent laws, what is claimed and desired protected is set forth in the appended claims.

## Claims

1. A system for accessing underlying tissue, which comprises:
an elongated access sheath defining a longitudinal axis; and
a penetrating tip releasably mounted to the access sheath and dimensioned for facilitating passage through tissue, the penetrating tip being removable through the access sheath, and an elongated removal member, **characterised in that** the elongated member is configured to be positionable within the access sheath to be mounted to the penetrating tip to permit the elongated removal member to remove the penetrating tip through the access sheath subsequent to passage of the penetrating tip through tissue, wherein the elongated removal member is releasably mounted to the penetrating tip, and wherein the elongated removal member and the penetrating tip includes cooperating structure for effecting releasable coupling of the elongated removal member to the penetrating tip.

2. The system according to claim 1 wherein the cooperating structure includes one of a bayonet coupling, threaded coupling, tongue and groove coupling and interference coupling.

3. The system according to claim 1 wherein the penetrating tip includes a transparent region adapted to permit passage of light.

4. The system according to claim 3 including a laparoscope having an illumination system for delivering illuminating light and an imaging system for detecting and transmitting an illuminated image of the surgical object, the laparoscope at least partially positionable within the access sheath to permit visualization during entry of the access sheath and the penetrating tip within the tissue.

5. The system according to claim 1 including a sheath housing mounted to the access sheath.

6. The system ·according to claim 5 wherein the sheath housing includes a valve adapted to form a substantial seal about an elongated object passed through the sheath housing.

## Patentansprüche

1. System zum Zugreifen auf ein tieferliegendes Gewebe, wobei das System umfasst:
eine längliche Zugangshülle, die eine Längsachse definiert; und
eine Penetrationsspitze, die lösbar an der Zugangshülle angebracht ist und hinsichtlich ihrer Größe eingerichtet ist, ein Hindurchdringen durch Gewebe zu erleichtern, wobei die Penetrationsspitze durch die Zugangshülle hindurch entfernbar ist, und ein längliches Entfernelement, **dadurch gekennzeichnet, dass** das längliche Element dafür eingerichtet ist, innerhalb der Zugangshülle positionierbar zu sein, um an der Penetrationsspitze angebracht werden zu können, um dem länglichen Entfernelement zu ermöglichen, die Penetrationsspitze durch die Zugangshülle zu entfernen, nachdem die Penetrationsspitze durch Gewebe gedrungen ist, wobei das längliche Entfernelement lösbar an der Penetrationsspitze angebracht ist, und wobei das längliche Entfernelement und die Penetrationsspitze eine Kooperationsstruktur aufweisen, um lösbares Koppeln des länglichen Entfernelements mit der Penetrationsspitze zu bewerkstelligen.

2. System gemäß Anspruch 1, bei dem die Kooperationsstruktur eines der Elemente Bajonettverbindung, Gewindeverbindung, Nut-Feder-Verbindung und Übermaßverbindung umfasst.

3. System gemäß Anspruch 1, bei dem die Penetrationsspitze einen transparenten Bereich aufweist, der dafür eingerichtet ist, ein Durchlassen von Licht zu erlauben.

4. System gemäß Anspruch 3, das ein Laparoskop mit einem Beleuchtungssystem zum Bereitstellen von Beleuchtungslicht und mit einem Abbildungssystem zum Detektieren und Übertragen eines beleuchteten Bildes des chirurgischen Objekts aufweist, wobei das Laparoskop wenigstens teilweise innerhalb der Zugangshülle positionierbar ist, um eine Visualisierung während des Eindringens der Zugangshülle und der Penetrationsspitze in das Gewebe zu erlauben.

5. System gemäß Anspruch 1, das ein Hüllengehäuse aufweist, das an der Zugangshülle angebracht ist.

6. System gemäß Anspruch 5, bei dem das Hüllengehäuse ein Ventil aufweist, das dafür eingerichtet ist, eine wesentliche Versiegelung um ein längliches Objekt, das durch das Hüllengehäuse hindurchgeführt ist, zu bilden.

## Revendications

1. Système d'accès à un tissu sous-jacent, qui comprend :
une gaine d'accès allongée définissant un axe longitudinal ; et
une pointe de pénétration montée de manière amovible sur la gaine d'accès et dimensionnée pour faciliter le passage à travers le tissu, la pointe de pénétration étant amovible à travers la gaine d'accès, et un élément d'enlèvement allongé, **caractérisé en ce que** l'élément allongé est configuré pour pouvoir être positionné dans la gaine d'accès à monter sur la pointe de pénétration afin de permettre à l'élément d'enlèvement allongé de retirer la pointe de pénétration à travers la gaine d'accès après le passage de la pointe de pénétration à travers le tissu, dans lequel l'élément d'enlèvement allongé est monté de manière amovible sur la pointe de pénétration et dans lequel l'élément d'enlèvement allongé et la pointe de pénétration comprennent une structure de coopération pour assurer un couplage libérable de l'élément d'enlèvement allongé avec la pointe de pénétration.

2. Système selon la revendication 1, dans lequel la structure de coopération comprend une structure choisie entre un couplage à baïonnette, un couplage vissé, un couplage à rainure et languette et un couplage à interférence.

3. Système selon la revendication 1, dans lequel la pointe de pénétration comprend une région transparente qui est à même de laisser passer la lumière.

4. Système selon la revendication 3, comprenant en outre un laparoscope ayant un système d'éclairage pour délivrer de la lumière d'éclairage et un système d'imagerie pour détecter et transmettre une image éclairée de l'objet chirurgical, le laparoscope pouvant au moins en partie être positionné dans la gaine d'accès pour permettre une visualisation au cours de l'entrée de la gaine d'accès et de la pointe de pénétration dans le tissu.

5. Système selon la revendication 1, comprenant un boîtier de gaine monté sur la gaine d'accès.

6. Système selon la revendication 5, dans lequel le boîtier de gaine comprend une soupape qui est à même de former un joint étanche sensible autour d'un objet allongé passé à travers le boîtier de gaine.
